# EUROPEAN PATENT SPECIFICATION

(11) **EP 1 996 640 B1**
(45) Date of publication and mention of the grant of the patent: **26.08.2009**
(21) Application number: 07723471.4
(22) Date of filing: 21.03.2007
(51) Int. Cl.: C08G 18/72, A61K 9/50

(54) **MICROPARTICLES COMPRISING A CROSSLINKED POLYMER**
MIKROPARTIKEL, UMFASSEND EIN VERNETZTES POLYMER
MICROPARTICULES COMPRENANT UN POLYMÈRE RÉTICULÉ

(30) Priority: 21.03.2006 EP 06075678
(43) Date of publication of application: 03.12.2008
(73) Proprietor: DSM IP Assets B.V., 6411 TH Heerlen (NL)
(72) Inventor: DIAS, Aylvin, Jorge, Angelo, Athanasius, NL-6213 HE Maastricht (NL); PETIT, Audrey, NL-6212 BJ Maastricht (NL)
(74) Representative: Vandevijver, Pascale
(86) International application number: PCT/EP2007/002514
(87) International publication number: WO 2007/107358

(56) References cited:
- EP-A- 1 172 693
- US-A1- 2005 013 869
- US-B1- 6 228 423
- US-B1- 6 787 597

## Description

The invention relates to microparticles comprising a crosslinked polymer, to a method of preparing such microparticles and to the use of the microparticles.

Spherical microparticles (microspheres) comprising crosslinked polymers are described in WO 98/22093. These microspheres are intended for use as a delivery system for a releasable compound (a drug). It is stated that the crosslinkable polymer used to prepare the particles is not critical. Suitable polymers mentioned in this publication are crosslinkable water-soluble dextrans, derivatized dextrans, starches, starch derivatives, cellulose, polyvinylpyrrolidone, proteins and derivatized proteins.

A disadvantage is that the pore size of the cross-linked polymer must be smaller than the particle size of the releasable compound. Thus, it is not possible to load the microspheres with the releasable compound after the microspheres have been made. It is therefore not possible to prepare a master batch of the microspheres without the releasable compound and to decide later which releasable compound to include in the microspheres.

It would however be desirable to be able to load microparticles afterwards, for instance because it would allow upscaling of the preparation process of the particles to provide a large batch of the particles, of which - if desired - different portions can be loaded with different active agents, in useful quantities for a specific purpose. Further, it would be desirable to be able to load microparticles afterwards in case an agent to be released from the microparticles may be detrimentally affected, e.g. degraded, denaturated or otherwise inactivated, during the preparation of the particles.

Microparticles, comprising non-crosslinked biodegradable polyesters are described in US-B-6.228.423. The polyesters comprise an amine group in the side chain. These microparticles are used as a carrier for a biologically active material, which is capable of eliciting an immune response.

Also US 2005/0013869 discloses microparticles for a sustained release formulation for a therapeutically active compound. The microparticles comprise non-crosslinked biodegradable polymers, in particular a polyester, poly (phosphate), poly(anhydride), poly(ortho-ester) or a mixture thereof. The therapeutically active compound is a carbamate, which is effective as an AChE inhibitor or binding agent.

The properties of known microparticles have been reported to be detrimentally affected as a result of aggressive processing for example freeze-drying. Especially in medical applications and in particular in drug delivery applications, good storage stability of the drug-loaded microparticles is important. A suitable method for providing long term product stability of drug delivery systems is lyophilisation (freeze-drying).

To counter the above problem of detrimentally affected microparticles, cryoprotectants are used in order to maintain the original microparticle characteristics such as size and shape, (See Saez et. al. European Journal of Pharmaceutics or Biopharmaceutics 50 (2000) 379-387, Chacon et. al. European Journal of Pharmaceutical Sciences, 8 (1999) 99-107).

There is a continuous need for alternative or improved microparticles comprising a crosslinked polymer. In particular, it would be desirable to provide a microparticle comprising a crosslinked polymer, which can be suitably processed under aggressive processing condition, with a low risk of being damaged to an unacceptable extent. Under aggressive processing conditions is in particular understood a condition that causes the particle to be subjected to a physical shock, such as a (fast) change in temperature for example a change of at least 1 °C per sec. - as happens in a freeze drying process or a sudden change in pressure, for example (repeated) pressurization and/or depressurization. For example in a pellet making machine use is made of a pressure of 0.5 T per cm² per sec.

It would further be desirable to provide a microparticle comprising a crosslinked polymer that can adequately be loaded with an active substance, such as a biologically active agent during microparticle formation and/or after the microparticle has been prepared.

Accordingly, it is an object of the present invention to provide a novel microparticle that can serve at least as an alternative to known microparticles and in particular to provide a microparticle that has a favourable property, such as showing good resistance against a physical shock.

Moreover it is an object of the present invention to provide a microparticle being efficiently loadable with an active agent.

Another object of the present invention is to provide a microparticle having one or more other favourable properties as identified herein below. It has been found to provide a microparticle comprising a crosslinked polymer which polymer is composed of a crosslinkable compound represented by the formula 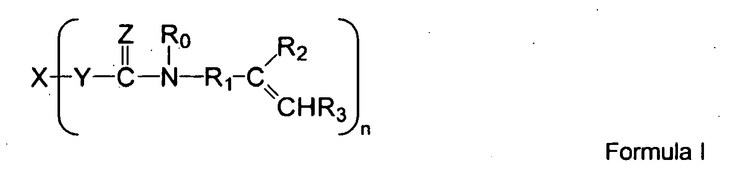 wherein
- X is a residue of a multifunctional radically polymerisable compound (having at least a functionality equal to n);
- each Y independently is optionally present, and - if present - each Y independently represents a moiety selected from the group of O, S and NR₀;
- each R₀ is independently chosen from the group of hydrogen and substituted and unsubstituted, aliphatic, cycloaliphatic and aromatic hydrocarbon groups which groups optionally contain one or more moieties selected from the group of ester moieties, ether moieties, thioester moieties, thioether moieties, carbamate moieties, thiocarbamate moieties, amide moieties and other moieties comprising one or more heteroatoms, in particular one or more heteroatoms selected from S, O, P and N, each R₀ in particular independently being chosen from the group of hydrogen and substituted and unsubstituted alkyl groups, which alkyl groups optionally contain one or more heteroatoms, in particular one or more heteroatoms selected from P, S, O and N;
~ each Z is independently chosen from O and S;
~ each R₁ is independently chosen from the group of substituted and unsubstituted, aliphatic, cycloaliphatic and aromatic hydrocarbon groups which groups optionally contain one or more moieties selected from the group of ester moieties, ether moieties, thioester moieties, thioether moieties, carbamate moieties, thiocarbamate moieties, amide moieties and other moieties comprising one or more heteroatoms, in particular one or more heteroatoms selected from S, O, P and N.;
~ each R₂ is independently chosen from hydrogen and substituted and unsubstituted, aliphatic, cycloaliphatic and aromatic hydrocarbon groups which groups optionally contain one or more moieties selected from the group of ester moieties, ether moieties, thioester moieties, thioether moieties, carbamate moieties, thiocarbamate moieties, amide moieties and other moieties comprising one or more heteroatoms, in particular one or more heteroatoms selected from S, O, P and N, each R₀ in particular independently being chosen from the group of hydrogen and substituted and unsubstituted alkyl groups, which alkyl groups optionally contain one or more heteroatoms, in particular one or more heteroatoms selected from P, S, O and N; and n is at least 2.
- each R₃ is chosen from hydrogen, -COOCH₃, -COOC₂H₅, -COOC₃H₇, -COOC₄H₉.

In particular R₀ is hydrogen or a hydrocarbon comprising up to 12 carbons. R₀ may be hydrogen or a substituted or unsubstituted C₁ to C₆ alkyl. R₀ may also be a substituted or unsubstituted cycloalkyl, more in particular a substituted or unsubstituted C₁ to C₃ alkyl or hydrogen. The cycloalkyl may be a cyclopentyl, cyclohexyl or cycloheptyl. The alkyl may be a linear or branched alkyl. A preferred branched alkyl is t-butyl.

Optionally R₀ may comprise a carbon-carbon double or triple bond, R₀ may for example comprise a -CH=CH₂ group.

R₀ may comprise an heteroatom, for example an ester moiety, such as -(C=O)-O-(CH₂)ᵢ-CH₃ or -(C=O)-O-(CH₂)ᵢ-CH=CH₂, wherein i is an integer, usually in the range of 0-8, preferably in the range of 1-6. The heteroatom may also be a ketomoiety, such as. -(C=O)-(CH₂)ᵢ-CH₃ or -(C=O)-(CH₂)ᵢ-CH=CH₂, wherein i is an integer, usually in the range of 0-8, preferably in the range of 1-6. An R₀ group comprising a heteroatom preferably comprises a NR'R" group, wherein R' and R" are independently a hydrogen or a hydrocarbon group, in particular a C1-C6 alkyl.

More preferred R₀ is hydrogen or an alkyl group. Still more preferably, R₀ is hydrogen or a methyl group.

Preferably R₁ comprises 1-20 carbon atoms. More preferably R₁ is a substituted or unsubstituted C₁ to C₂₀ alkylene, in particular a substituted or unsubstituted C₂ to C₁₄ alkylene. R₁ may comprise an aromatic moiety, such as o-phenylene, m-phenylene or p-phenylene. The aromatic moiety may be unsubstituted or substituted, for instance with an amide, for example an acetamide.

R₁ may comprise a - (O-C=O)-, a -(N-C=O), a - (O-C=S)-functionality. It is also possible that R₁ comprises an alicyclic moiety, for example a cyclopentylene, cyclohexylene or a cycloheptylene moiety, which optionally comprises one or more heteroatoms for example a N-group and/or a keto-group.

Optionally R₁ comprises a carbon-carbon double or triple bond, in particular R₁ may comprise a -CH=CH₂ group.
In a preferred embodiment R₁ is chosen from a -CH₂-CH₂-O-C(O)-, -CH₂-CH₂-N-C(O)- or -CH2-CH₂-O-C(S)- group.

R₂ is for example hydrogen or a hydrocarbon comprising up to 12 carbons. In particular R₂ may be hydrogen or a substituted or unsubstituted C₁ to C₆ alkyl, more in particular a substituted or unsubstituted C₁ to C₃ alkyl.
Optionally R₂ comprises a carbon-carbon double or triple bond, in particular R₂ may comprise a -CH=CH₂ group. n is preferably 2-8.

R3 is preferably hydrogen.

Substituents on R₀, R₁ and/or R₂ may for example be chosen from halogen atoms and hydroxyl. A preferred substituent is hydroxyl. In particular R₁ is a - CH₂OH group because it is commercially available.

The polymer is generally cross-linked via reaction of vinylic bonds of the compound shown in Formula I.

### LEGEND TO THE FIGURES

Figure 1 shows a SEM photograph of microparticles according to the invention.

Figure 2 shows a size distribution of a plurality of microparticles according to the invention.

Figure 3 shows a release profile of microparticles according to the invention, loaded with a drug.

Advantageously, the microparticle, which may be a microsphere, in particular in case if the crosslinked polymer is a carbamate, thiocarbamate, a ureyl or an amide copolymer, is tough but still elastic. This is considered beneficial with respect to allowing processing under aggressive conditions, such as sudden pressure changes, high temperatures, low temperatures and/or conditions involving high shear.

The microparticles of the present invention show a good resistance against a sudden decrease in temperature, which may for example occur if the microparticles are lyophilised.

In a preferred embodiment, the microparticles according to the present invention are even essentially free of cryoprotectants. A cryoprotectant is a substance that protects a material, i.c.microparticles, from freezing damage (damage due to ice formation). Examples of cryoprotectants include a glycol, such as ethylene glycol, propylene glycol and glycerol or dimethyl sulfoxide (DMSO).

It is further envisaged that the microparticles of the present invention show a good resistance against heating, which may occur if the particles are sterilised (at temperatures above 120°C) or if the particles are loaded with an active substance at elevated temperatures for example temperatures above 100 °C.

The microparticles of the present invention may be used as a delivery system for an active agent, in particular a drug, a diagnostic aid or an imaging aid. The microparticles can also be used to fill a capsule or tube by using high pressure or may be compressed as a pellet, without substantially damaging the microparticles. It can also be used in injectable or spray-able form as a suspension in a free form or in an in-situ forming gel formulation. Furthermore, the microparticles can be incorporated in for example (rapid prototyped) scaffolds, coatings, patches, composite materials, gels or plasters.
The microparticle according to the present invention can be injected, sprayed, implanted or absorbed.

Y in formula I is optionally present, and - if present - each Y independently represents a moiety selected from the group of O, S and NR₀.

X in formula I is a residue of a multifunctional radically polymerisable compound, preferably X is a residue of a -OH, -NH₂, -RNH or -SH multifunctional polymer or oligomer. The multifunctional polymer or oligomer is in particular selected from biostable or biodegradable polymers or oligomers that can be natural or synthetic.

The term biodegradable refers to materials that experience degradation by hydrolysis or by the action of an enzyme or by the action of biological agents present in their environment such as bacteria and fungi. Such may be attributable to a microorganism and/or it may occur in the body of an animal or a human.

The term biostable refers to materials which are not substantially broken down in a biological environment, in case of an implant at least not noticeably within a typical life span of a subject, in particular a human, wherein the implant has been implanted.

Examples of biodegradable polymers are polylactide (PLA); polyglycolide (PGA), polydioxanone, poly(lactide-co-glycolide), poly(glycolide-co-polydioxanone), polyanhydrides, poly (glycolide-co-trimethylene carbonate), poly(glycolide-co-caprolactone), poly- (trimethylenecarbonates), aliphatic polyesters, poly(orthoesters); poly (hydroxyl-acids), polyamino-carbonates or poly(ε-caprolactones) (PCL).

Examples of biostable or synthetic polymers are poly (urethanes); poly (vinyl alcohols) (PVA); polyethers, such as poly alkylene glycols, preferably poly (ethylene glycols) (PEG); polythioethers, aromatic polyesters, aromatic thioesters, polyalkylene oxides, preferably selected from poly (ethylene oxides) and poly (propylene oxides); poloxamers, meroxapols, poloxamines, polycarbonates, poly (vinyl pyrrolidones): poly (ethyl oxazolines).

Examples of natural polymers are polypeptides, polysaccharides for example polysucrose, hyaluronic acid, dextran and derivates thereof, heparin sulfate, chondroitin sulfate, heparin, alginate, and proteins such as gelatin, collagen, albumin, ovalbumin, starch, carboxymethylcellulose or hydroxyalkylated cellulose and co-oligomers, copolymers, and blends thereof.

X in formula I may be chosen based upon its biostability/ biodegradability properties. For providing microparticles with high biostability polyethers, polythioethers, aromatic polyesters or aromatic thioesters are generally particularly suitable. For providing microparticles with high biodegradability aliphatic polyesters, aliphatic polythioesters, aliphatic polyamides, aliphatic polycarbonates or polypeptides are particularly suitable. Preferably X is selected from an aliphatic polyester, aliphatic polythioester, aliphatic polythioether, aliphatic polyether or polypeptide.More preferred are copolymersor blends comprising PLA, PGA, PLGA, PCL and/or poly (ethylene oxide)-co-poly (propylene oxide) block co-oligomers/copolymers.

A combination of two or more different moieties forming X may be used to adapt the degradation rate of the particles and/or the release rate of an active agent loaded in or on the particles, without having to change the particle size, although of course one may vary the particle size, if desired. The two or more different moieties forming X are for example a copolymer or co-oligomer (i.e. a polymer respectively oligomer comprising two or more different monomeric residues). A combination of two or more different moieties forming X may further be used to alter the loading capacity, change a mechanical property and/or the hydrophilicity/hydrophobicity of the microparticles.

The (number average) molecular weight of the X-moiety is usually chosen in the range of 100 to 100 000 g/mol. In particular, the (number average) molecular weight may be at least 200, at least 500, at least 700 or at least 1 000 g/mol. In particular, the (number average) molecular weight may be up to 50 000 or up to 10 000 g/mol. In the present invention the (number average) molecular weight is as determinable by size exclusion chromatography (GPC), using the method as described in the Examples.

In a preferred embodiment, the X-moiety in the cross-linked polymer is based on a compound having at least two functionalities that can react with an isocyanate to form a carbamate, thiocarbamate or ureyl link. In such an embodiment, the Y group is present in formula I. The X moiety is usually a polymeric or oligomeric compound with a minimum of two reactive groups, such as hydroxyl (-OH), amine or thiol groups.

In another embodiment, X is the residue of a amine-bearing compound to provide an alkenoyl urea, providing a compound represented by the formula, X-(N-CO-NR-CO-CH=CH₂)ₙ or X-(N-CO-NR-CO-C(CH₃)=CH2)ₙ). Examples thereof are in particular poly(propenoylurea), poly(methylpropenoylurea) or poly(butenoylurea). Herein each R independently represents a hydrocarbon group such as identified above.

In still another embodiment, X is the residue of a thiol-bearing compound to provide a compound represented by the formula X-(S-C(S)-NH-Phenyl-CH=CH₂)₂, such as a poly(alkenyl carbamodithioic) ester.

In a further embodiment, X is the residue of a carboxylic acid bearing compound to provide a compound represented by the formula X-(C(O)-NR-C(O)-CH=CH2)ₙ. Herein each R independently represents a hydrocarbon group such as identified above. An example thereof is poly((methyl-)oxo-propenamide.

As used in this application, the term "oligomer" in particular means a molecule essentially consisting of a small plurality of units derived, actually or conceptually, from molecules of lower relative molecular mass. It is to be noted that a molecule is regarded as having an intermediate relative molecular mass if it has properties which vary significantly with the removal of one or a few of the units. It is also to be noted that, if a part or the whole of the molecule has an intermediate relative molecular mass and essentially comprises a small plurality of the units derived, actually or conceptually, from molecules of lower relative molecular mass, it may be described as oligomeric, or by oligomer used adjectivally. In general, oligomers have a molecular weight of more than 200 Da, such as more than 400, 800, 1000, 1200, 2000, 3000, or more than 4000 Da. The upper limit is defined by what is defined as the lower limit for the mass of polymers (see next paragraph).

Accordingly the term "polymer" denotes a structure that essentially comprises a multiple repetition of units derived, actually or conceptually, from molecules of low relative molecular mass. Such polymers may include crosslinked networks, branched polymers and linear polymers. It is to be noted that in many cases, especially for synthetic polymers, a molecule can be regarded as having a high relative molecular mass if the addition or removal of one or a few of the units has a negligible effect on the molecular properties. This statement fails in the case of certain macromolecules for which the properties may be critically dependant on fine details of the molecular structure. It is also to be noted that, if a part or the whole of the molecule has a high relative molecular mass and essentially comprises the multiple repetition of units derived, actually or conceptually, from molecules of low relative molecular mass, it may be described as either macromolecular or polymeric, or by polymer used adjectivally. In general, polymers have a molecular weight of more than 8000 Da, such as more than 10.000, 12.000, 15.000, 25.000, 40.000, 100.000 or more than 1.000.000 Da.

Microparticles have been defined and classified in various different ways depending on their specific structure, size, or composition, see e.g. Encyclopaedia of Controlled drug delivery Vol2 M-Z Index, Chapter: Microencapsulation Wiley Interscience, starting at page 493, see in particular page 495 and 496.

As used herein, microparticles include micro- or nanoscale particles which are typically composed of solid or semi-solid materials and which are capable of carrying an active agent. Typically, the average diameter of the microparticles given by the Fraunhofer theory in volume percent ranges from 10 nm to 1000 µm. The preferred average diameter depends on the intended use. For instance, in case the microparticles are intended for use as an injectable drug delivery system, in particular as an intravascular drug delivery system, an average diameter of up to 10 µm, in particular of 1 to 10 µm may be desired.

It is envisaged that microparticles with a average diameter of less than 800 nm, in particular of 500 nm or less, are useful for intracellular purposes. For such purposes, the average diameter preferably is at least 20 nm or at least 30 nm. In other applications, larger dimensions may be desirable, for instance a diameter in the range of 1-100 µm or 10-100 µm. In particular, the particle diameter as used herein is the diameter as determinable by a LST 230 Series Laser Diffraction Particle size analyzer (Beckman Coulter), making use of a UHMW-PE (0.02 - 0.04 µm) as a standard. Particle-size distributions are estimated from Fraunhofer diffraction data and given in volume (%).

If the particles are too small or non analyzable by light scattering because of their optical properties then scanning electron microscopy (SEM) or transmission electron microscopy (TEM) can be used.

Several types of microparticle structures can be prepared according to the present invention. These include substantially homogenous structures, including nano- and microspheres and the like. However in case that more than one active agent has to be released or in case that one or more functionalities are needed it is preferred that the microparticles are provided with a structure comprising an inner core and an outer shell. A core/shell structure enables more multiple mode of action for example in in drug delivery of incompatible compounds or in imaging. The shell can be applied after formation of the core using a spray drier. The core and the shell may comprise the same or different crosslinked polymers with different active agents. In this case it is possible to release the active agents at different rates. It is also possible that the active agent is only present in the core and that the shell is composed of crosslinked polymers capable to provide lubricity.

In a further embodiment the microparticles may comprise a core comprising the crosslinked polymers according to the present invention and a shell comprising a magnetic or magnetisable material.

In still a further embodiment, the microparticles may comprise a magnetic or magnetisable core and a shell comprising the crosslinked polymers according to the present invention. Suitable magnetic or magnetisable materials are known in the art. Such microparticles may be useful for the capability to be attracted by objects comprising metal, in particular steel, for instance an implanted object such as a graft or a stent. Such microparticles may further be useful for purification or for analytical purposes.

In a still further embodiment, the particles are imageable by a specific technique. Suitable imaging techniques are MRI, CT, X-ray. The imaging agent can be incorporated inside the particles or coupled onto their surface. Such particles may be useful to visualize how the particles migrate, for instance in the blood or in cells. A suitable imaging agent is for example gadolinium.

The microparticles according to the present invention may carry one or more active agents. An active agent may be more or less homogeneously dispersed within the microparticles or within the microparticle core. The active compound may also be located within the microparticle shell.

In particular, the active agent may be selected from the group of nutrients, pharmaceuticals, proteins and peptides, vaccines, genetic materials, (such as polynucleotides, oligonucleotides, plasmids, DNA and RNA), diagnostic agents, and imaging agents. The active agent, such as an active pharmacologic ingredient (API), may demonstrate any kind of activity, depending on the intended use.
The active agent may be capable of stimulating or suppressing a biological response. The active agent may for example be chosen from growth factors (VEGF, FGF, MCP-1, PIGF, antibiotics (for instance penicillin's such as B-lactams, chloramphenicol), antiinflammatory compounds, antithrombogenic compounds, anti-claudication drugs; anti-arrhythmic drugs, anti-atherosclerotic drugs, antihistamines, cancer drugs, vascular drugs, ophthalmic drugs, amino acids, vitamins, hormones, neurotransmitters, neurohormones, enzymes, signalling molecules and psychoactive medicaments.

Examples of specific active agents or drugs are neurological drugs (amphetamine, methylphenidate), alpha1 adrenoceptor antagonist (prazosin, terazosin, doxazosin, ketenserin, urapidil), alpha2 blockers (arginine, nitroglycerin), hypotensive (clonidine, methyldopa, moxonidine, hydralazine minoxidil), bradykinin, angiotensin receptor blockers (benazepril, captopril, cilazepril, enalapril, fosinopril, lisinopril, perindopril, quinapril, ramipril, trandolapril, zofenopril), angiotensin-1 blockers (candesartan, eprosartan, irbesartan, losartan, telmisartan, valsartan), endopeptidase (omapatrilate), beta2 agonists (acebutolol, atenolol, bisoprolol, celiprolol, esmodol, metoprolol, nebivolol, betaxolol), beta2 blockers (carvedilol, labetalol, oxprenolol, pindolol, propanolol) diuretic actives (chlortalidon, chlorothiazide, epitizide, hydrochlorthiazide, indapamide, amiloride, triamterene), calcium channel blockers (amlodipin, bamidipin, diltiazem, felodipin, isradipin, lacidipin, lercanidipin, nicardipin, nifedipin, nimodipin, nitrendipin, verapamil), anti arthymic active (amiodarone, solatol, diclofenac, enalapril, flecainide) or ciprofloxacin, latanoprost, flucloxacillin, rapamycin and analogues and limus derivatives, paclitaxel, taxol, cyclosporine, heparin, corticosteroids (triamcinolone acetonide, dexamethasone, fluocinolone acetonide), anti-angiogenic (iRNA, VEGF antagonists: bevacizumab, ranibizumab, pegaptanib), growth factor, zinc finger transcription factor, triclosan, insulin, salbutamol, oestrogen, norcantharidin, microlidil analogues, prostaglandins, statins, chondroitinase, diketopiperazines, macrocycli compounds, neuregulins, osteopontin, alkaloids, immuno suppressants, antibodies, avidin, biotin, clonazepam.

The active agent can be delivered for local delivery or as pre or post surgical therapies for the management of pain, osteomyelitis, osteosarcoma, joint infection, macular degeneration, diabetic eye, diabetes mellitus, psoriasis, ulcers, atherosclerosis, claudication, thrombosis viral infection, cancer or in the treatment of hernia.

In accordance with the present invention, if an active agent is present, the concentration of one or more active agent in the microparticles, is preferably at least 5 wt. %, based on the total weight of the microparticles, in particular at least 10 wt. %, more in particular at least 20 wt. %. The concentration may be up to 90 wt. %, up to 70 wt. %, up to 50 wt. % or up to 30 wt. %, as desired.

The fields wherein microparticles according to the present invention can be used include dermatology, vascular, orthopedics, ophthalmic, spinal, intestinal, pulmonary, nasal, or auricular.

Besides in a pharmaceutical application, microparticles according to the invention may inter alia be used in an agricultural application. In particular, such microparticles may comprise a pesticide or a plant-nutrient.

It is also possible to functionalise at least the surface of the microparticles by providing at least the surface with a functional group, in particular with a signalling molecule, an enzyme or a receptor molecule, such as an antibody. The receptor molecule may for instance be a receptor molecule for a component of interest, which is to be purified or detected, e.g. as part of a diagnostic test, making use of the particles of the present invention. Suitable functionalisation methods may be based on a method known in the art. In particular, the receptor molecule may be bound to the crosslinked polymer of which the particles are composed, via a reactive moiety in the residue X. An example of a reactive moiety in residue X is a carbodiimide group or a succinamide group

If the microparticles for example comprise -OH and/or -COOH groups, for example in the X-moiety it is possible to functionalize such an -OH or - COOH group with a carbodiimide which may further react with a hydroxyl group of a target functional moiety to be coupled to the particles.

To couple a target functional moiety comprising an amide group N-hydroxysuccinimide (NHS) may be used. In particular NHS may be coupled to the microparticles if the microparticles comprise a polyalkylene glycol moiety, such as a PEG moiety. Such polyalkylene glycol moiety may in particular be the X residue or part thereof as presented in Formula I.

A target functional moiety may also comprise an -SH group, for example a cysteine residue which may be coupled to the microparticles by first reacting the microparticles with vinyl sulfone. In particular vinyl sulfone may be coupled to the microparticles if the microparticles comprise a polyalkylene glycol moiety, such as a PEG moiety. Such polyalkylene glycol moiety may in particular be the X group or part thereof as presented in Formula I. Various other coupling agents are known, (See Fisher et. al. Journal of Controlled release 111 (2006) 135-144 and Kasturi et.al. Journal of Controlled release 113 (2006) 261-270.

In principle microparticles may be prepared in a manner known in the art, provided that the polymers used in the prior art are (at least partially) replaced by the crosslinkable compound of formula I.

In addition to the cross-linkable compound represented by formula I, the microparticles of the present invention may further comprise one or more other compounds selected from the group of polymers and cross-linkable or polymerisable compounds. The polymers may in particular be polymers such as described above. The crosslinkable or polymerisable compounds may in particular be compounds selected from the group of acrylic compounds and other olefinically unsaturated compounds, for example, vinyl ether, allylether, allylurethane, fumarate, maleate, itaconate or unsaturated acrylate units. Suitable unsaturated acrylates are, for example, unsaturated urethaneacrylates, unsaturated polyesteracrylates, unsaturated epoxyacrylates and unsaturated polyetheracrylates.

The other polymers or polymerisable compounds may be used to adjust a property of the microparticles, for example to tune the release profile of an active agent or to obtain a complete polymerization (i.e. no residual reactive unsaturated bonds that may be cytotoxic) or to narrow the size distribution of the microparticle. In case, the microparticles are prepared from a combination of the compound of Formula I and one or more other polymerisable compounds, crosslinked polymers may be formed, composed of both the compound of Formula I and the one or more other compounds.

The weight to weight ratio of the group of other polymers and polymerisable compounds to the compound represented by Formula I may be 0 or more. If another polymer or polymerisable compound is present, the ratio of the group of other polymers and polymerisable compounds to the compound represented by Formula I usually is at least 10:90, in particular at least 25:75 or at least 45:55. Preferably, the ratio is 90:10 or less, in particular 55:45 or less or 35:65 or less.

The microparticle is for example prepared by the steps of
- reacting the multifunctional radically polymerisable compound X with an isocyanate represented by the formula II. 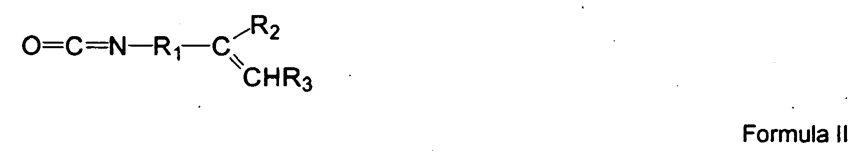 wherein X, R₁ R₂ and R₃ are as defined herein above;
~ forming droplets comprising the reaction product (represented by formula I)
~ and cross-linking the reaction product.
   An advantage of such method is its simplicity whereby the microparticle can be prepared starting from only two starting materials: a compound providing X and the compound of Formula II, especially for compounds of Formula II that are commercially available.

An alternative preparation route is via the reaction: 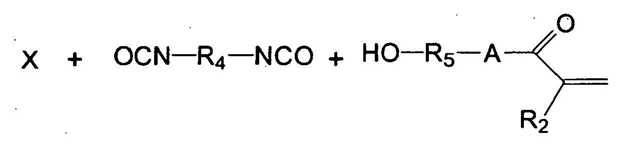 wherein R₄ is an aliphatic, cycloaliphatic or aromatic group, wherein R₅ is an alkyl (C2-C4), wherein A is chosen from O or N and R₂ is as defined in formula I.
Such alternative preparation method is advantageous for practical reasons, especially in terms of ease of commercially obtaining raw materials with various R-groups. Instead of an isocyanate also a thioisocyanate can be used.

The droplets are preferably formed by making an emulsion comprising the reaction product in a discontinuous phase. The compound of Formula I may be emulsified in for example water, an aqueous solution or another liquid or solvent. The stability of the emulsion may be enhanced by using known surfactant, for example triton X, polyethylene glycol or Tween 80. Using emulsion polymerisation is simple and is in particular suitable for a batch-process.

It is also possible to prepare the droplets making use of extrusion, spray drying or ink jet technology. Herein, a liquid comprising the reaction product is extruded or "jetted", typically making use of a nozzle, into a suitable gas, e.g. air, nitrogen, a noble gas or the like, or into a non-solvent for the liquid and the reaction product. The size of the droplets can be controlled by the viscosity of the formulation, the use of a vibrating nozzle and/or a nozzle where a electrical filed is applied. By selecting a suitable temperature for the non-solvent or the gas and/or by applying another condition, e.g. radiation, crosslinking is accomplished, thereby forming the microparticles of the invention, e.g. as described in Espesito et al., Pharm. Dev, Technol 5(2);267-278 or Ozeki et. al. Journal of controlled release 107 (2005) 387-394. Such process is in particular suitable to be carried out continuously, which may in particular be advantageous in case large volumes of the microparticles are to be prepared.

The reaction temperature is usually above the melting temperature of the compound of Formula I. It is also an option to dissolve the compound in a solvent, below or above the melting temperature of the compound. Besides allowing forming the droplets at a relatively low temperature, this may be useful in order to prepare porous particles. It is also possible to use a reactive solvent, for example a solvent that may react with the polymerising reagents, for instance as solvent that is a radically polymerisable monomer. In this way a fine tuning of the network density of the microparticle can be achieved. The temperature is generally below the boiling temperature of the liquid phase(s).

Cross-linking may be carried out in any suitable way known for cross-linking compounds comprising vinyl groups, in particular by thermal initiation (aided by a thermo initiator, such as a peroxide or an azo-initatior, e.g. azobisisobutylonitrile), by photo-initiation (aided by a photo-initiator such as a Norrish type I or II initiator), by redox-initiation, or any (other) mechanism that generates radicals making use of a chemical compound and/ or electromagnetic radiation. Examples of suitable crosslinkers are trimethylolpropane trimethacrylate, diethylene glycol dimethacrylate or Hydroxyethylacrylate.

If desired the microparticles may be loaded with one or more active agents. Loading may be achieved by forming the microparticles in the presence of the active agent or thereafter. To achieve microparticles with a high amount of active agent, it is generally preferred to prepare the microparticles in the presence of the active agent. In particular in the case that the active agent is sensitive to the cross-linking or may adversely affect or interfere directly or indirectly with the cross-linking, it is preferred to load the microparticles after they have been formed. This can be achieved by contacting the microparticles with the active agent and allowing the agent to diffuse into the particles and/or adhere/ adsorb to the surface thereof.

In accordance with the invention it is possible to provide microparticles with one or more active agents with satisfactory encapsulation efficiency. (i.e. the amount of active agent in the particles, divided by the amount of active agent used). Depending upon the loading conditions, an efficiency of at least about 50 %, at least about 75 % or at least 90 % or more is feasible.

The invention will now be illustrated by the following examples without being limited thereto.

### Materials and Methods

Poly(ethylene glycol) 35 kD (PEG), Tin (II) ethylhexonoate, Peroxodisulphate (KPS), terazosin hydrochloride, diethylene glycol dimethacrylate(DEGDMA), trimethylolpropane trimethacrylate (TMPTMA), Irgacure 819. Polycaprolactone triol (PCL₃₀₀), Hydroxyethylacrylate (HEA), 2,4-toluenedi-isocyanate (TDI) and Darocur 1173 were purchased from Sigma-Aldrich. PTGL₁₀₀₀ (i.e. Poly(-methyl-1,4-butanediol)co(tetramethyleneglycol), having an Mw of 1000 g/mol) was from Desotech, Isocyanate ethylmethacrylate (IEMA) was purchased from KarenzMOI (purity: 98%). Irganox 1035 was from Ciba Speciality Chemicals. The chemicals were used as such unless otherwise stated.

Nuclear Magnetic Resonance (NMR) experiments were performed on a Varian Inova 300 spectrometer.

Infrared experiments were performed on a Perkin Elmer Spectrum FT-IR Spectrometer 1760 x, 1720 x. The polymer samples were placed between two KBr tablets.

Acrylate conversion measured were performed on a Perkin Elmer Spectrum One FTIR spectrometer equipped with a Golden Gate attenuated total reflection (ATR) accessory was used. The spectrum One consists of a DTGS detector and the Golden Gate making use of a single bounce diamond crystal. Infrared spectra between 4000 and 650 cm⁻¹ were recorded averaging 4 scans with a spectral resolution of 4 cm⁻¹. The transmission spectra were transformed in absorption spectra. The peak height was determined at 1410, 1630, and, 810 cm⁻¹ to measure acrylate conversion.

Size Exclusion Chromatography (SEC) was performed using a Waters 515 HPLC pump, a Waters 410 Differential Refractometer and a Servern Analytical SA6503 Programmable Absorbance Detector equipped with a Waters Styragel HR 2,3 and 4 column at flow rate of 1 ml/min using tetrahydrofuran (THF) as the eluent. SEC data were obtained using the IR detector. The system was calibrated using narrow polystyrene standards (EasyCal PS2, from Polymer Laboratories, Heerlen).

All experiments related to terazosin concentration measurement were done by liquid chromatography (duplot measurement for TRH). The HLPC system (HP 1090 Liquid Chromatograph) consisted of the following components: DR5 pump, diode array detector (DAD), built-in autosampler, and ChemStation software, version Rev. A. 08.03 (Agilent Technologies). A C18 analytical column 150 x 4.6 mm (XTerra RP 18, Waters) with a mean particle size of 3.5 µm was used at 40.0 oC. Flow rates were 1.5 ml/min. A mobile phase gradient composed of mobile phase A, 10 mM phosphate buffer, and B, acetonitrile, was used. The eluent gradients were as follows: during one gradient cycle of 14 min, the mobile phase was changed from 10 to 95 % of mobile phase B over a period of 8 min, kept at 95 % mobile phase B for 2 min and thereafter, lowered to 10 % of mobile phase B in 4 min, where it was kept until the next sample was injected. The injection volume was 50 µl. The detection was done at 250 and 340 nm.

LST 230 Series Laser Diffraction Particle size analyzer (Beckman Coulter) was used to measure size distribution of the microparticles. The standard was UHMwPE (0.02 - 0.04 µm).

A Leica DMLB microscope (magnitude x 50 to x 400) was used to analyse the morphology of the microspheres.

A Philips CP SEM XL30 at an accelerating voltage of 5 and 10 kV was used to examine the microparticles. The specimens were mounted in a SEM sample holder and a conductive Au-layer was applied (2*60 s, 20mA).

### Example 1: Synthesis of PTGL₁₀₀₀-(IEMA)₂ oligomer

51 mg (0.1 wt. % based on total weight) of Irganox 1035, 13.9 g (0.09 mol) of IEMA and 11 mg (0.1 mol % with respect to the IEMA) of tin(II) 2-ethyl hexanoate were stirred together in a 100-ml reaction flask under dry air. 45.6 g (0.045 mol) of PTGL₁₀₀₀ was added drop wise over 30 min. at a constant temperature (20 °C). Next, the reaction mixture was heated to 60°C and allowed to proceed for 18 h. The formation of PTGL₁₀₀₀-(IEMA)₂ was validated with the following analytical results: ¹H-NMR (300 MHz, CDCl₃, 22 °C): δ (ppm) = 6.26-5.83 (s, 1H, H-CH=CH(CH₃)-); 5.78-5.77 (s, 1H, H-CH=CH(CH₃)-); 4.50-4.05 (m, 2H, -O-CH₂-CH₂-NH-); 4.05-3.88 (m,2H,-O-CH₂-CH₂-NH-); 3.22-2.51 (m, 2H, -O-CH₂-CH₂-CH(CH₃)-); 1.95-4.79 (s, 3H, CH2=CH(CH₃)-); 1.66-1.38 (s, 24H, -CH₂-CH(CH₃)-CH₂-); IR (neat, cm-1): 1723.59 (C=O, stretch), 1638.14 (C=C); SEC (IR detector): M_{w}= 4800, PDI = 1.57.

### Example 2: Preparation of PTGL₁₀₀₀-(IEMA)₂ microspheres

2 g PTGL₁₀₀₀-(IEMA)₂ oligomer and 20 g of a PEG solution (20% in demi-water) were stirred at 1500 rpm (Eurostar Power Control Visc, IKA-WERKE) for 15 min at 60°C. The stirring was stopped to let the emulsion stabilize. After 15 min, 4.5 ml of KPS solution (50 mg/ml) was added. The polymerization was allowed to proceed for two hours at 70°C. The microspheres were isolated by centrifugation (Harrier 15/80, MSE, 15 min at 4500 rpm) and washed with 20 ml of demi-water. The morphology of the microspheres was checked by microscopy. The particle size analyzer gave an average diameter of 25 µm (d₇₅/d₂₅ = 9).

### Example 3: Synthesis of PTGL₁₀₀₀-(TDI-HEA)₂

75.48 g (0.65 mol) HEA was added drop wise to 113.20 g (0.65 mol) TDI in the presence of 0.3 g (0.48 mmol) or tin II ethyl hexanoate (0.5g (1.3 mmol). The conversion of the isocyanate groups (NCO) was monitored by a titration. 174.95 g (0.60 mol) of this HEA-TDI mixture was added to 301.33 grams PTGL1000 from Hodogaya (0.60 mol OH) and 0.3 g Irganox 1035 and stirred. The temperature was gradually increased to 80°C. After 7 hours the NCO value was 0.026 %. Overnight the reaction mixture cooled down till 50°C. After another 16 hours the NCO level was 0.007%. The yield of the urethane diacrylate oligomer was 450 g (92%.)

### Example 4: Preparation of PTGL₁₀₀₀-(TDI-HEA)₂ microspheres

1.70 g PTGL₁₀₀₀-(TDI-HEA)₂ oligomer and 15 g of a PEG solution (20% in demi-water) were stirred at 1500 rpm (Eurostar Power Control Visc, IKA-WERKE) for 15 min at RT. The stirring was stopped to let the emulsion stabilize. After 15 min, 5 ml of aqueous KPS solution (50 mg/ml) were added. The emulsion was stirred for 10 min at 500 rpm. The polymerization was allowed to proceed for two hours at 70°C. The microspheres were isolated by centrifugation (Harrier 15/80, MSE, 15 min at 4500 rpm) and washed twice with 20 ml of demi-water. The morphology of the microspheres was checked by microscopy. The particle size analyzer gave an average diameter of 130 µm (d₇₅/d₂₅ = 7).

### Example 5: Preparation of PTGL₁₀₀₀-(IEMA)₂/EGDMA/TMPTMA microparticles

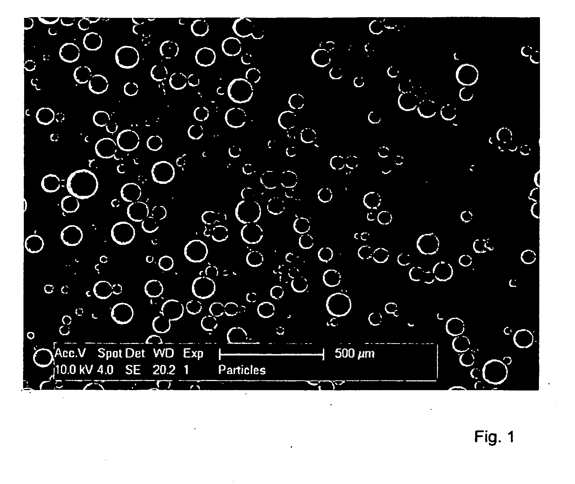

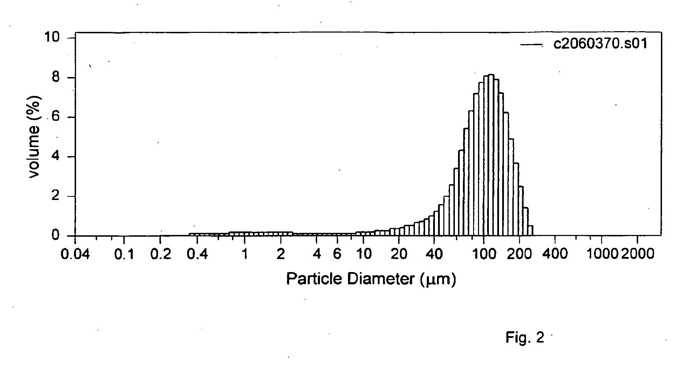

A formulation was prepared with 1.4 g PTGL₁₀₀₀ (IEMA)₂, 0.5 g DEGDMA, 0.1 g TMPTMA and 20 mg of Darocur 1173. An aqueous solution was prepared with 2 g of PEG and 13 g of demi-water. To the aqueous solution, the formulation was added to give an emulsion. The emulsion was stirred for 30 min at 500 rpm (Heidolph MR3002). The polymerization was allowed to proceed for 30 min, under UV light (Macam Flexicure controller, D-bulb, 200 mW/s/cm2). After polymerisation, the microparticles were isolated by centrifugation (Harrier 15/80, MSE, 15 min at 4500 rpm) and washed twice with 20 ml of demi-water. The morphology of the microparticles was checked by scanning electron microscopy (see Figure 1). The particle size analyzer gave an average diameter of 100 µm (d₇₅/d₂₅=1.9) (see Figure 2). The acrylate conversion was 80%.

### Example 6 Preparation of functional PTGL₁₀₀₀-(TDI-IEMA)₂ microparticles

A formulation was prepared with 1.5 g PTGL₁₀₀₀(TD)-HEA)₂, 1.5 g HEA and 30 mg of Irgacure 819. An aqueous solution was prepared with 4 g of PEG and 21 g of demi-water. The formulation was added drop-wise into the aqueous solution to give an emulsion. The emulsion was stirred for 30 min at 500 rpm (Heidolph MR3002). The polymerisation was allowed to proceed for 30 min under UV light (Macam Flexicure controller, D-bulb, 200 mW/s/cm2). After polymerisation, the microparticles were isolated by centrifugation (Harrier 15/80, MSE, 15 min at 4500 rpm) and washed twice with 20 ml of demi-water. The morphology of the microparticles was checked by microscopy. The particle size analyzer gave an average diameter of 390 µm (d₇₅/d₂₅=2.5). The acrylate conversion was superior to 95%. These microparticles are composed of hydroxyl groups that can further be used for functionalization.

### Example 7 Release profile of PTGL₁₀₀₀-(IEMA)₂/EGDMA/TMPTMA microparticles

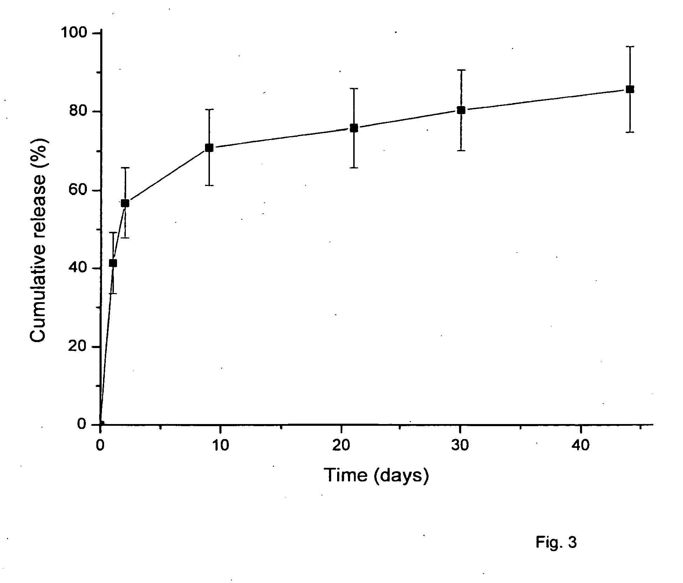

Three batches of 100 mg of dried microparticles (from Example 5) were incubated with 2 ml of a terazosin solution (5 mg/ml in phosphate buffered saline (PBS)). This resulted in a loading of 10 %. Water was evaporated overnight in an oven at 60°C. The dried microparticles were washed three times with 7.5 ml of PBS. The concentration of terazosin in the washing steps was determined to determine the encapsulation efficiency. The encapsulation efficiency was 75 %. The release profile was studied in PBS at 37°C. The results are shown in Figure 3. The vertical bars show the standard deviation (n=3).

### Example 8: Freeze-drying stability of PTGL₁₀₀₀-(TDI-HEA)₂/HEA microparticles

Microparticles of Example 6 were freeze dried (Edwards Freeze dryer Micro Modulyo equipped with a vacuum pump Edwards 5 two stages and a pressure controller Vaccuubrand CVC2) overnight. After reconstitution in demi-water, the morphology of the microparticle was checked by microscopy (no broken microparticles were observed). The particle size analyser gave an average diameter of 360 µm, which represents a deviation of less than 7 % compared to the diameter measured with fresh microparticles. This illustrates that these microparticles show good resistance against a detrimental effect (a reduction in size), as a result of a physical shock (freeze drying).

### Example 9: Pressure stability of PTGL-based microparticles

The microparticles of Example 5 were compressed using a KBr press. A pressure of 5 tons was maintained for 5 minutes. After reconstitution in demi-water, the morphology of the microparticles was checked by microscopy. No broken microparticles were observed. The particle size analyzer gave an average diameter of 110 µm, which represents a deviation of only 10 %, compared to the diameter measured with microparticles not subjected to compression.

### Example 10: Preparation of on-fly PTGL₁₀₀₀-(TDI-HEA)₂ microparticles

1.5 g PTGL₁₀₀₀-(TDI-HEA)₂, 1.5 g HEA and 30 mg of Irgacure 819 were mixed together. This formulation was dripped in the air through a needle of 0.6 mm diameter. While falling through the air, the microparticles were UV polymerized (using a Macam Flexicure controller, D-bulb, 200 mW/s/cm2) and collected in ethylene glycol. A post curing of micropartcles in ethylene glycol was performed for 30 min. The morphology and the size of the microparticles were estimated by microscopy: the average diameter was 1000 µm with a narrow distribution (950 - 1050 µm, visually determined, using a microscope).

### Example 11: Synthesis of PCL₃₀₀-IEMA₃

Polycaprolacton triol (80 gram, 0.266 mol), Irganox 1035 (0.2 gram, 0.1 w% wrt the total weight) were stirred for 10 min. IEMA (124 gram, 0.800 mol) was added drop wise in 90 min. The reaction mixture was heated to 60°C and stirred for 4 hours upon the reaction was complete as indicated by IR and NMR. ¹H-NMR (300 MHz, CDCl₃, 22°C, TMS): δ (ppm) = 6.1 (CH, methacrylate), 5.6 (CH, methacrylate), 5.0 (NH. urethane), 4,2 (2H, -CH₂-CH₂-), 4.0 (CH2-CO-), 3.5 (2H,-CH₂-CH₂-), 2.4 (CH3-CH2), 1.4-1.7 (6H, -CH2-CH2-CH2-), 1.9 3H (CH3, methacrylate)-CH2), 0.9 3H (CH3-CH2).

### Example 12: Preparation of biodegradable PCL ₃₀₀-IEMA₃ microparticles

1 g of PCL300-IEMA3 was mixed with 1 g PEG , 6.5 g demi-water and 70 mg Darocur 1173 for 15 min (Heidolph MR3002, 1250 rpm). The polymerisation was allowed to proceed for 60 min under UV light (Macam Flexicure controller, D-bulb, 200 mW/s/cm2). After polymerization, the microparticles were filtered through a 0.8 µm filter under vacuum (Gelman Sciences Supor-800) and rinse with 100 ml demi-water. The morphology was checked with light microscopy. The acrylate conversion was 90 %. The average size was 140 µm (D75/D25 = 3.2).

## Claims

1. Microparticle comprising a crosslinked polymer, which polymer is composed of a crosslinkable compound represented by the formula 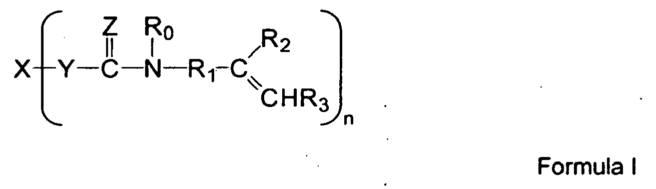 wherein
~ X is a residue of a multifunctional radically polymerisable compound (having at least a functionality equal to n);
~ each Y independently is optionally present, and - if present - each Y independently represents a moiety selected from the group of O, S and NR₀;
~ each R₀ is independently chosen from the group of hydrogen and substituted and unsubstituted, aliphatic, cycloaliphatic and aromatic hydrocarbon groups which groups optionally contain one or more moieties selected from the group of ester moieties, ether moieties, thioester moieties, thioether moieties, carbamate moieties, thiocarbamate moieties, amide moieties and other moieties comprising one or more heteroatoms, in particular one or more heteroatoms selected from S, O, P and N, each R₀ in particular independently being chosen from the group of hydrogen and substituted and unsubstituted alkyl groups, which alkyl groups optionally contain one or more heteroatoms, in particular one or more heteroatoms selected from P, S, O and N;
- each Z is independently chosen from O and S;
~ each R₁ is independently chosen from the group of substituted and unsubstituted, aliphatic, cycloaliphatic and aromatic hydrocarbon groups which groups optionally contain one or more moieties selected from the group of ester moieties, ether moieties, thioester moieties, thioether moieties, carbamate moieties, thiocarbamate moieties, amide moieties and other moieties comprising one or more heteroatoms, in particular one or more heteroatoms selected from S, O, P and N.;
- each R₂ is independently chosen from hydrogen and substituted and unsubstituted, aliphatic, cycloaliphatic and aromatic hydrocarbon groups which groups optionally contain one or more moieties selected from the group of ester moieties, ether moieties, thioester moieties, thioether moieties, carbamate moieties, thiocarbamate moieties, amide moieties and other moieties comprising one or more heteroatoms, in particular one or more heteroatoms selected from S, O, P and N, each R₀ in particular independently being chosen from the group of hydrogen and substituted and unsubstituted alkyl groups, which alkyl groups optionally contain one or more heteroatoms, in particular one or more heteroatoms selected from P, S, O and N; and n is at least 2,
- each R₃ is chosen from hydrogen, -COOCH₃, -COOC₂H₅, -COOC₃H₇, - COOC₄H₉.R.

2. Microparticle according to claim 1, wherein X is the residue of a OH, -NH₂, -RNH or -SH multifunctional polymer or oligomer.

3. Microparticle according to any one of claims 1-2, wherein X is selected from a biostable or biodegradable polymer or oligomer.

4. Microparticle according to claim 3, wherein X is selected from an aliphatic polyester, aliphatic polythioester, aliphatic polythioetiher, aliphatic polyether or polypeptide.

5. Microparticle according to any one of the claims 1-4 wherein R₀ is hydrogen or an alkyl group.

6. Microparticle according to any one of the preceding claims wherein R₁ comprises 2-20 carbon atoms, preferably 2-14 carbon atoms.

7. Microparticle according to any one of the preceding claims wherein R₂ is hydrogen or comprises 1-6 carbon atoms.

8. Microparticle according to any one of the preceding claims, wherein the average diameter is in the range of 10 nm to 1000 µm, preferably in the range of 1-100 µm.

9. Microparticle according to any one of the preceding claims wherein the microparticles are provided with a structure comprising an inner core and an outer shell.

10. Microparticle according to any one of the preceding claims comprising one or more active agents.

11. Microparticle according to claim 10, wherein the active agent is selected from the group of nutrients, pharmaceuticals, proteins and peptides, vaccines, genetic materials, diagnostic agents or imaging agents.

12. Microparticle according any one of the preceding claims, wherein the crosslinked polymer is a carbamate, thiocarbamate, ureyl or amide copolymer.

13. Method for preparing a microparticle according to any one of the preceding claims, comprising the steps of
- reacting a multifunctional radically polymerisable compound X with an isocyanate represented by formula II 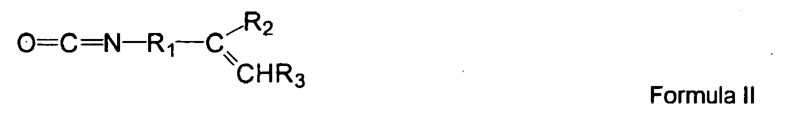 wherein X, R₁, R₂ and R₃ are as defined in claim 1,
~ making droplets comprising the reaction product;
~ and cross-linking the reaction product.

14. Use of a microparticle according to any one of the claims 1-12 as a delivery system for an active compound, in particular a drug, a diagnostic aid or an imaging aid.

15. Use of the microparticle according to claim 14 in dermatology, vascular, orthopedics, ophthalmic, spinal, intestinal, pulmonary, nasal, or auricular.

16. Use of the microparticle according to any one of claims 14-15 in suspensions, capsules, tubes, pellets, (rapid prototyped) scaffolds, coatings, patches, composite materials or plasters or (in situ forming) gels..

17. Use of the microparticle according to claim 16 whereby the microparticle can be injected, sprayed, implanted or absorbed.

## Patentansprüche

1. Mikroteilchen, umfassend ein vernetztes Polymer, welches Polymer aus einer vernetzbaren Verbindung folgender Formel zusammengesetzt ist, 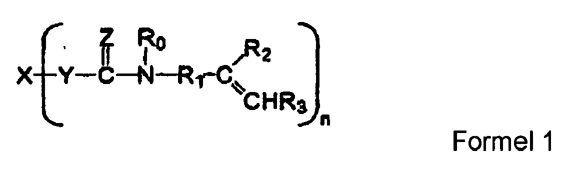 wobei
- X ein Rest einer multifunktionellen, radikalisch polymerisierbaren Verbindung (mit einer Funktionalität von wenigstens n) ist;
- jedes Y unabhängig gegebenenfalls vorhanden ist und, falls vorhanden, jedes Y unabhängig eine Einheit, ausgewählt aus der Gruppe von O, S und NR₀, darstellt;
- jedes R₀ unabhängig ausgewählt ist aus der Gruppe von Wasserstoff und substituierten und unsubstituierten aliphatischen, cycloaliphatischen und aromatischen Kohlenwasserstoffresten, welche Reste gegebenenfalls eine oder mehrere Einheiten umfassen, ausgewählt aus der Gruppe von Estereinheiten, Ethereinheiten, Thioestereinheiten, Thioethereinheiten, Carbamateinheiten, Thiocarbamateinheiten, Amideinheiten und anderen Einheiten, die ein oder mehrere Heteroatome umfassen, insbesondere ein oder mehrere Heteroatome, die aus S, O, P und N ausgewählt sind, wobei jedes R₀ insbesondere unabhängig ausgewählt ist aus der Gruppe von Wasserstoff und substituierten und unsubstituierten Alkylresten, welche Alkylreste gegebenenfalls ein oder mehrere Heteroatome umfassen, insbesondere ein oder mehrere Heteroatome, die aus P, S, O und N ausgewählt sind;
- jedes Z unabhängig aus O und S ausgewählt ist;
- jedes R₁ unabhängig ausgewählt ist aus der Gruppe von substituierten und unsubstituierten aliphatischen, cycloaliphatischen und aromatischen Kohlenwasserstoffresten, welche Reste gegebenenfalls eine oder mehrere Einheiten umfassen, ausgewählt aus der Gruppe von Estereinheiten, Ethereinheiten, Thioestereinheiten, Thioethereinheiten, Carbamateinheiten, Thiocarbamateinheiten, Amideinheiten und anderen Einheiten, die ein oder mehrere Heteroatome umfassen, insbesondere ein oder mehrere Heteroatome, die aus S, O, P und N ausgewählt sind;
- jedes R₂ unabhängig ausgewählt ist aus Wasserstoff und substituierten und unsubstituierten aliphatischen, cycloaliphatischen und aromatischen Kohlenwasserstoffresten, welche Reste gegebenenfalls eine oder mehrere Einheiten umfassen, ausgewählt aus der Gruppe von Estereinheiten, Ethereinheiten, Thioestereinheiten, Thioethereinheiten, Carbamateinheiten, Thiocarbamateinheiten, Amideinheiten und anderen Einheiten, die ein oder mehrere Heteroatome umfassen, insbesondere ein oder mehrere Heteroatome, die aus S, O, P und N ausgewählt sind, wobei jedes R₀ insbesondere unabhängig ausgewählt ist aus der Gruppe von Wasserstoff und substituierten und unsubstituierten Alkylresten, welche Alkylreste gegebenenfalls ein oder mehrere Heteroatome umfassen, insbesondere ein oder mehrere Heteroatome, die aus P, S, O und N ausgewählt sind; und n wenigstens 2 ist;
- jedes R₃ ausgewählt ist aus Wasserstoff, -COOCH₃, -COOC₂H₅, -COOC₃H₇, -COOC₄H₉, R.

2. Mikroteilchen gemäß Anspruch 1, wobei X der Rest eines OH-, -NH₂-, -RNH- oder -SH-multifunktionellen Polymers oder Oligomers ist.

3. Mikroteilchen gemäß einem der Ansprüche 1-2, wobei X aus einem biologisch stabilen oder biologisch abbaubaren Polymer oder Oligomer ausgewählt ist.

4. Mikroteilchen gemäß Anspruch 3, wobei X aus einem aliphatischen Polyester, aliphatischen Polythioester, aliphatischen Polythioether, aliphatischen Polyether und einem Polypeptid ausgewählt ist.

5. Mikroteilchen gemäß einem der Ansprüche 1-4, wobei R₀ Wasserstoff oder ein Alkylrest ist.

6. Mikroteilchen gemäß einem der vorstehenden Ansprüche, wobei R₁ 2-20 Kohlenstoffatome umfasst, vorzugsweise 2-14 Kohlenstoffatome.

7. Mikroteilchen gemäß einem der vorstehenden Ansprüche, wobei R₂ Wasserstoff ist oder 1-6 Kohlenstoffatome umfasst.

8. Mikroteilchen gemäß einem der vorstehenden Ansprüche, wobei der mittlere Durchmesser im Bereich von 10 nm bis 1000 µm liegt, vorzugsweise im Bereich von 1-100 µm.

9. Mikroteilchen gemäß einem der vorstehenden Ansprüche, wobei die Mikroteilchen mit einer Struktur dargeboten sind, die einen inneren Kern und eine äußere Hülle umfasst.

10. Mikroteilchen gemäß einem der vorstehenden Ansprüche, umfassend einen oder mehrere Wirkstoffe.

11. Mikroteilchen gemäß Anspruch 10, wobei der Wirkstoff aus der Gruppe von Nährstoffen, Pharmazeutika, Proteinen und Peptiden, Impfstoffen, genetischen Materialien, diagnostischen Mitteln und Bildgebungsmitteln ausgewählt ist.

12. Mikroteilchen gemäß einem der vorstehenden Ansprüche, wobei das vernetzte Polymer ein Carbamat-, Thiocarbamat-, Ureyl- oder Amid-Copolymer ist.

13. Verfahren zum Herstellen eines Mikroteilchens gemäß einem der vorstehenden Ansprüche, umfassend folgende Schritte:
- Umsetzen einer multifunktionellen, radikalisch polymerisierbaren Verbindung X mit einem Isocyanat der Formel II, 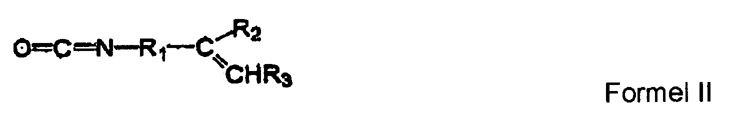
wobei X, R₁, R₂ und R₃ wie in Anspruch 1 definiert sind;
- Herstellen von Tröpfchen, die das Reaktionsprodukt umfassen;
- und Vernetzen des Reaktionsprodukts.

14. Verwendung eines Mikroteilchens gemäß einem der Ansprüche 1-12 als Abgabesystem für einen Wirkstoff, insbesondere ein Arzneimittel, ein Diagnosehilfsmittel oder ein Bildgebungshilfsmittel.

15. Verwendung des Mikroteilchens gemäß Anspruch 14 in der Dermatologie, vaskulär, der Orthopädie, ophthalmisch, spinal, intestinal, pulmonär, nasal oder aurikulär.

16. Verwendung des Mikroteilchens gemäß einem der Ansprüche 14-15 in Suspensionen, Kapseln, Tuben, Pellets, (durch schnelles Prototyping erhaltenen) Gerüsten, Beschichtungen, Pflastern, Kompositmaterialien oder plastischen Massen oder (in situ bildenden) Gelen.

17. Verwendung des Mikroteilchens gemäß Anspruch 16, wobei das Mikroteilchen injiziert, versprüht, implantiert oder absorbiert werden kann.

## Revendications

1. Microparticule comprenant un polymère réticulé, lequel polymère est constitué d'un composé réticulable représenté par la formule 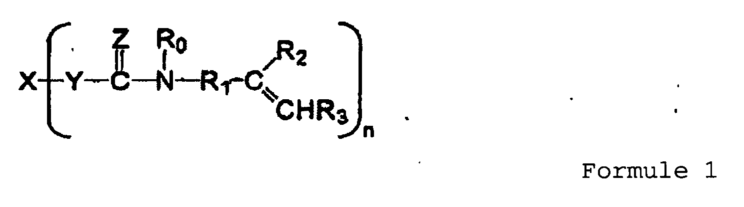 dans laquelle
- X est un résidu d'un composé multifonctionnel polymérisable par voie radicalaire (ayant au moins une fonctionnalité égale à n) ;
- chaque Y indépendamment est éventuellement présent, et - s'il est présent - chaque Y représente indépendamment un motif choisi dans le groupe de O, S et NR₀ ;
- chaque R₀ est choisi indépendamment dans le groupe d'hydrogène et de groupements hydrocarbonés aliphatiques, cycloaliphatiques et aromatiques, substitués et non substitués, lesquels groupements contiennent éventuellement un ou plusieurs motifs choisis dans le groupe de motifs ester, de motifs éther, de motifs thioester, de motifs thioéther, de motifs carbamate, de motifs thiocarbamate, de motifs amide et d'autres motifs comprenant un ou plusieurs hétéroatomes, en particulier un ou plusieurs hétéroatomes choisis parmi S, O, P et N, chaque R₀ en particulier indépendamment étant choisi dans le groupe d'hydrogène et de groupements alkyle substitués et non substitués, lesquels groupements alkyle contiennent éventuellement un ou plusieurs hétéroatomes, en particulier un ou plusieurs hétéroatomes choisis parmi P, S, O et N ;
- chaque Z est choisi indépendamment parmi O et S ;
- chaque R₁ est choisi indépendamment dans le groupe de groupements hydrocarbonés aliphatiques, cycloaliphatiques et aromatiques, substitués et non substitués, lesquels groupements contiennent éventuellement un ou plusieurs motifs choisis dans le groupe de motifs ester, de motifs éther, de motifs thioester, de motifs thioéther, de motifs carbamate, de motifs thiocarbamate, de motifs amide et d'autres motifs comprenant un ou plusieurs hétéroatomes, en particulier un ou plusieurs hétéroatomes choisis parmi S, O, P et N ;
- chaque R₂ est choisi indépendamment parmi hydrogène et des groupements hydrocarbonés aliphatiques, cycloaliphatiques et aromatiques, substitués et non substitués, lesquels groupements contiennent éventuellement un ou plusieurs motifs choisis dans le groupe de motifs ester, de motifs éther, de motifs thioester, de motifs thioéther, de motifs carbamate, de motifs thiocarbamate, de motifs amide et d'autres motifs comprenant un ou plusieurs hétéroatomes, en particulier un ou plusieurs hétéroatomes choisis parmi S, O, P et N, chaque R₀ en particulier indépendamment étant choisi dans le groupe d'hydrogène et de groupements alkyle substitués et non substitués, lesquels groupements alkyle contiennent éventuellement un ou plusieurs hétéroatomes, en particulier un ou plusieurs hétéroatomes choisis parmi P, S, O et N ; et n est au moins 2,
- chaque R₃ est choisi parmi hydrogène, -COOCH₃, - COOC_{2H5}, -COOC₃H₇, -COOC₄H₉, R.

2. Microparticule selon la revendication 1, **caractérisée en ce que** x est le résidu d'un oligomère ou d'un polymère multifonctionnel OH, - NH₂, -RNH ou -SH.

3. Microparticule selon l'une ou l'autre des revendications 1 et 2, **caractérisée en ce que** X est choisi parmi un oligomère ou un polymère biostable ou biodégradable.

4. Microparticule selon la revendication 3, **caractérisée en ce que** X est choisi parmi un polyester aliphatique, un polythioester aliphatique, un polythioéther aliphatique, un polyéther aliphatique ou un polypeptide.

5. Microparticule selon l'une quelconque des revendications 1-4, **caractérisée en ce que** R₀ est hydrogène ou un groupement alkyle.

6. Microparticule selon l'une quelconque des revendications précédentes, **caractérisée en ce que** R₁ comprend 2-20 atomes de carbone, de préférence 2-14 atomes de carbone.

7. Microparticule selon l'une quelconque des revendications précédentes, **caractérisée en ce que** R₂ est hydrogène ou comprend 1-6 atomes de carbone.

8. Microparticule selon l'une quelconque des revendications précédentes, **caractérisée en ce que** le diamètre moyen est dans la gamme de 10 nm à 1000 µm, de préférence dans la gamme de 1-100 µm.

9. Microparticule selon l'une quelconque des revendications précédentes, **caractérisée en ce que** les microparticules sont proposées avec une structure comprenant un coeur interne et une coque externe.

10. Microparticule selon l'une quelconque des revendications précédentes, comprenant un ou plusieurs agents actifs.

11. Microparticule selon la revendication 10, **caractérisée en ce que** l'agent actif est choisi dans le groupe de nutriments, d'agents pharmaceutiques, de protéines et peptides, de vaccins, de matériaux génétiques, d'agents de diagnostic et d'agents d'imagerie.

12. Microparticule selon l'une quelconque des revendications précédentes, **caractérisée en ce que** le polymère réticulé est un copolymère de carbamate, de thiocarbamate, d'uréyle et d'amide.

13. Méthode de préparation d'une microparticule selon l'une quelconque des revendications précédentes, comprenant les étapes consistant à
- faire réagir un composé X multifonctionnel polymérisable par voie radicalaire avec un isocyanate représenté par la formule II 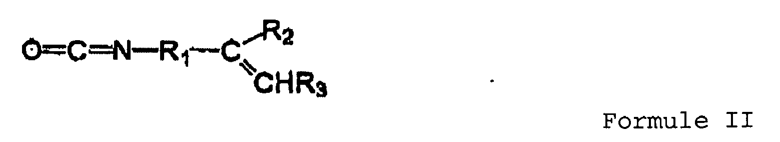
dans laquelle X, R₁, R₂ et R₃ sont tels que définis dans la revendication 1,
- préparer des gouttelettes comprenant le produit de réaction ;
- et réticuler le produit de réaction.

14. Utilisation d'une microparticule selon l'une quelconque des revendications 1-12 comme système de délivrance d'un composé actif, en particulier d'un médicament, d'un auxiliaire de diagnostic ou d'un auxiliaire d'imagerie.

15. Utilisation de la microparticule selon la revendication 14, dans le domaine dermatologie vasculaire, orthopédique, ophtalmique, spinal, intestinal, pulmonaire, nasal ou auriculaire.

16. Utilisation de la microparticule selon l'une ou l'autre des revendications 14 et 15, dans des suspensions, des capsules, des tubes, des pastilles, des échafaudages (prototypes rapides), des enrobages, des timbres, des matériaux composites ou des pansements adhésifs ou des gels (se formant in situ).

17. Utilisation de la microparticule selon la revendication 16, **caractérisée en ce que** la microparticule peut être injectée, pulvérisée, implantée ou absorbée.
